# EUROPEAN PATENT SPECIFICATION

(11) **EP 2 837 679 B1**
(45) Date of publication and mention of the grant of the patent: **01.05.2019**
(21) Application number: 14002847.3
(22) Date of filing: 14.08.2014
(51) Int. Cl.: C12M 1/107, A01C 3/02

(54) **Process and plant for removing nitrogen from poultry manure**
Verfahren und Anlage zur Beseitigung von Stickstoff aus Geflügeldung
Procédé et installation pour éliminer l'azote du fumier de volaille

(30) Priority: 16.08.2013 IT TS20130003
(43) Date of publication of application: 18.02.2015
(73) Proprietor: IGP s.r.l., 34147 Trieste (IT)
(72) Inventor: Migliardi, Daniela, 37132 Verona (IT); Tosto, Massimo, 34134 Trieste (IT); Vecchiet, Massimo, 34121 Trieste (IT)
(74) Representative: Ferraiolo, Ruggero

(56) References cited:
- WO-A1-2013/060338
- DE-A1- 10 353 728
- US-A1- 2013 047 852

## Description

### Field of the invention.

The field in which is the invention is that of the pre-treatment of the livestock waste, in particular manure produced by laying hens or other poultry, and other similar substrates for composition, in combination with the subsequent digestion of the same for the disposal of wastewater treated and / or for the production of biogas.

### Purpose of the invention.

Purpose of the present invention is to remove the nitrogen in substrates with high nitrogen content as pollen produced by laying hens or other poultry and / or by other similar substrates for composition, so as to eliminate the toxicity for the purpose of anaerobic digestion.

### State of the art.

Document DE 103 53 728 A1 discloses an anaerobic treatment where the complete transformation into biogas of slurry and manure happens in a time shorter than the usual 20 days in the conventional anaerobic treatments. The heart of the treatment is an anaerobic digester (sized for retention times of biomass under treatement longer than 10 days), combined with a post-treatment and recirculation of the digestate so as to accelerate the process of methanation.

Document US 2013/0047852 A1 discloses a post-treatment method for removing ammonia or reducing ammonium from biogas plant fermentation liquids or biogas plant fermentation residues wherein ammonia or ammonium is reduced by flash evaporation of a residual biomass of a conventional anaerobic digestion.

From the literature searching on the scientific publications on the production of biogas from manure (organic manure obtained from poultry manure) emerges as a major difficulty for its anaerobic treatment of the high concentration of ammonia nitrogen (> 3 g / 1), which involves onset of toxicity to methanogenic bacteria. Several approaches have been over the years aimed at reducing nitrogen from this matrix, also from experiences on an experimental scale, as well as anaerobic digestion plants in scale.

The most common strategy is the dilution of the droppings with water or with other substrates to limited nitrogen content and high moisture content. This solution, however, entails, on the one hand, the need of finding the necessary quantities of co-enzymes, the other due to high costs for the necessary volumes of digestion and storage and for the management of the digested product.

Other experiences reported in the scientific literature regarding treatments in laboratory scale, targeted to the removal / separation of the excess nitrogen, including: chemical precipitation of struvite (Demeestere et al., 2001), treatment with zeolite (Milan et al2001, Tada et al., 2005), Annamox process of nitrification and denitrification (Dong & Tollner, 2003), stripping of the digestate (Lei et al., 2007), stripping on leaching of manure (Gangagni Rao et el., 2008) and stripping the biogas product in dry fermentation of manure (Abouelenien et al., 2010).

The search for patent prior art showed a prevalence of proposals aimed nitrogen removal (such as reverse osmosis EP2215241B1, distillation in EP1683766B1), primarily downstream of the anaerobic digestion process. These solutions do not have points of contact with the present invention, since it does not provide for processes of increase of ammoniacal and increase of 'efficiency of the removal process, but is restricted to the removal of the nitrogen by filtration and / or distillation.

Document EP1478600A4 instead reveals the mixing of manure and digestate to make the mix pumpable, filtration to obtain a liquid fraction enriched in ammoniacal nitrogen, its heating at 65 ÷ 110 ° C, stripping, cooling and, finally, the reunification the solid fraction in the anaerobic digester. Also in this case, therefore, it is proposed the stripping of ammonia, however, in the absence of biological processes (hydrolysis) aimed to the increase of the ammonia and of chemical and physical processes (removal of CO2 with consequent raising of pH values) acts to the increase the efficiency of the stripping of NH3 that is the essence of our invention.

Document EP1929024B1 reveals the mixing of manure and digestate treated to make the mix pumpable and reveals a two-stage anaerobic digestion (hydrolysis / acidogenesis + methanogenesis). The digestate is subsequently split in two lines, of which a part is recirculated to the preparation of the substrate upstream of the process, and the second line is heated, stripped (75 ° ÷ 90 ° C) and further subjected to mechanical separation; the liquid is made.to flow in the head in the process and the solid is dried and removed from the system. The treatment therefore proposes the reduction of ammonia downstream of the anaerobic digestion process, using the same digestate as a means of dilution of manure and ammonia nitrogen. This configuration uses anaerobic digestion to promote mineralization and the formation of ammonia. In this process the nitrogen content of manure in input to the process does not, before the step of anaerobic digestion, a reduction in the mass, but only a reduction in the concentration (by dilution with digestate); only after the anaerobic digestion taking place the stripping of the digestate during which removes nitrogen.

### Drawbacks in the state of the art.

The process referred to in the document EP1929024B1 therefore differs from the treatment of the present proposal, which is designed as a pre-treatment in order to reduce nitrogen from substrates prior to entry in digestion, thus enabling the use in digestion of substrates modified with the reduction of the nitrogen content.

The document EP 2208712 relates to a plant for the production of biogas, where the steps of hydrolysis, of acidogenesis / acetogenesis and methanogenesis, occur in separate reactors. The hydrolysis is expected in aerobic conditions with oxygen injection. Downstream of the hydrolysis step involving a section of the ammonia nitrogen removal with reverse osmosis system. The purpose of this system is the optimization of the process of anaerobic digestion for production of biogas, the removal of ammoniacal nitrogen is not a goal, and if the request is achieved by a step of membrane filtration.

The state of the art reveals several techniques for nitrogen reduction, however none of these reveals a system composed of thermal pretreatment in two stages (a first at temperatures typical of the hydrolysis / acidogenesis followed by a second of overheating) with the purpose, in the hydrolytic stage, part of mineralizing organic nitrogen into ammonia and in the stage of overheating to alter the degree of solubility of CO2 with a consequent increase in pH, a condition that shifts the equilibrium toward the free and NH3 in the gaseous form, in the expense of ammonium ion (NH4 +) present in solution.

### The process object of the invention

In general, the essence of the invention is to implement a thermal pretreatment in two stages to favor the transformation of organic nitrogen contained in organic substrates having elevated concentrations of total nitrogen, ammoniacal nitrogen and create favorable conditions for the stripping and the removal of ammonia.

In particular, the procedure implements a sequence of biological processes and chemical-physical applied to organic substrates having elevated concentrations of total nitrogen for nitrogen removal at the same end of a use of said substrates in anaerobic digestion, as listed briefly below:
a) feeding of the substrate in the reactor thermal pretreatment (or in two reactors in series)
b) first heating the substrate at a controlled temperature and between 20 and 55 ° C, for sufficient time to make happen phenomena of hydrolysis / acidogenesis (a few days);
c) subsequent heating of the substrate hydrolyzed (output from phase b) by raising the temperature up to 65 ÷ 95 ° C with CO2 and insolubilization of the alteration of carbonates / bicarbonates in the substrate process and alkalinization of the substrate up to values equal or higher than 9 with consequent displacement of the chemical equilibrium ammonium / ammonia (NH4 + / NH3) towards the formation of free NH3 in gaseous form.
d) Stripping and nitrogen removal, typically by injection of air into the substrate,.

### Detailed description of the invention.

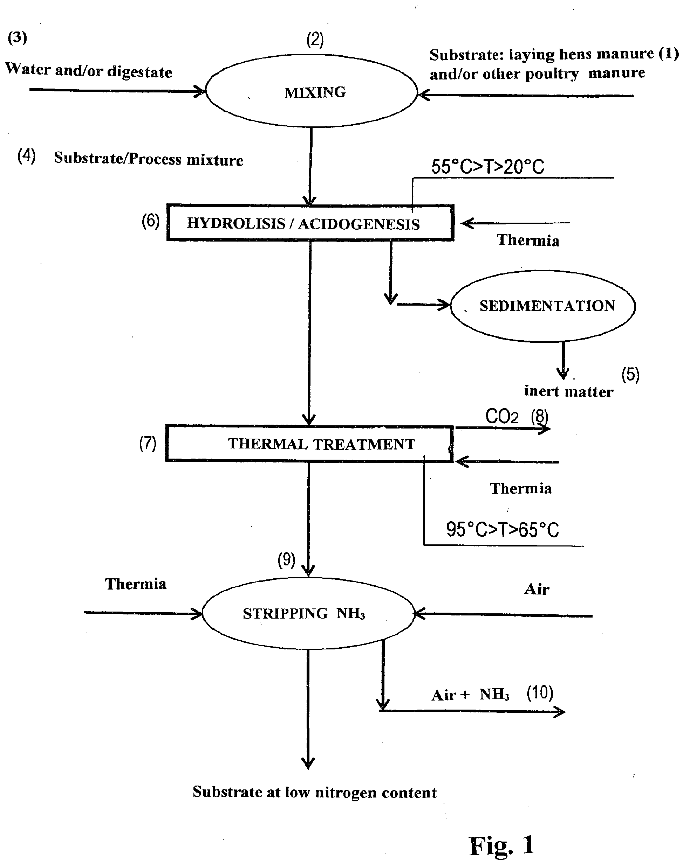

The invention will now be described in more detail with the aid of a drawing showing the basic components of a system suitable for the purpose and in which the - Fig. 1 is a diagram of the procedure.

The droppings (1) is produced daily and continuously in the intensive farming of laying hens and / or other poultry. The fresh droppings presents with pasty consistency and dense, and with a dry content of between 15 and 30%. In addition to high concentrations of nitrogen, the droppings of laying hens contains a high amount of calcium carbonate (coarse-grained and fine), added to the feed itself to aid digestion and the formation of the eggshell.

The substrate (1) (manure and / or other organic substrate with a high content of nitrogen) can also be mixed (2) with water and / or slurry, and / or digestate (3) in order to obtain a pumpable mixture (4) and promote the processes of hydrolysis and acidogenesis. Such operation of dilution makes easier the separation of substances with different specific weight (5).

The procedure starts with the feeding of the substrate (4) in the reactor in which it is initially heated to a temperature between 20 and 55 ° C to be the process of hydrolysis / acidogenesis (6), which takes place at a controlled temperature and constant. In the course of hydrolysis / acidogenesis (6) the complex organic matter is transformed by hydrolytic microorganisms into simpler compounds (simple sugars, proteins, amino acids, lipids, glycerol, free fatty acids), the hydrolysis products after being processed by fermenting microbiota in volatile organic acids (eg. acetic acid, propionic, butyric, valeric, caproic acid, etc..). Among the products "inorganic" released after hydrolysis of organic matter, include ammonia nitrogen (NH4 + NH3), carbon dioxide (CO2) and hydrogen sulfide (H2S = H + HS). As regards the nitrogen present in the organic substance, it is contained mainly in the amino groups of proteins and in the excretion of metabolites (eg. Uric acid as in the case of manure of poultry). At the end of the hydrolysis the organic nitrogen is mineralized and released in the aqueous phase as ammonium ion (NH4 +) or as free ammonia (NH3), according to the following chemical equilibrium:

NH3 (g) + H2O ↔ NH3 (SOLUTION) + H2O ↔ NH4 + + OH-

The phase of hydrolysis / acidogenesis (6) is also the time at which occurs the greater production of CO2. Given the relatively low temperature maintained at this stage (between 20 and 55 ° C), the CO2 produced remains predominantly dissolved in the process mixture; being a gas, its solubility is inversely proportional to the temperature of the liquid in which it is dissolved.

After the step of hydrolysis / acidogenesis (6), which normally lasts for a couple of days, it passes to the second heat treatment step (7), during which (in the same reactor or in a separate reactor in series to the first) is heating the substrate, up to values of temperature comprised between 65 and 95 ° C. The applied temperature difference causes the reduction of the solubility of CO2 in the mixture process. It follows a leakage of gas (8) from the liquid phase, and therefore the reduction of the concentration of dissolved CO2, which in turn leads to an alteration of the chemical equilibrium of carbonates/bicarbonates that, as final result, leads to an increased pH from the initial values to values equal to or greater than 9.

The increase of temperature and pH play an important role in the chemical ammonia. As shown by the following formula, the increase of pH and temperature favor the presence of NH3 free, to the detriment of the ammonium ion. The ammonia in the aqueous phase thus formed, always for the effect of heat, can undergo a change of state, passing from the liquid phase to the gaseous phase (NH3 (water) <-> NH3 (gas)).

### TAN=totalammonianitrogen.

Given the conditions of high volatility NH3 free, you can proceed with its removal from the substrate by stripping (9). By stripping (9) it is possible to remove the ammonia nitrogen in the liquid in the gaseous form (10).

The air and / or gas stripping enriched in NH3 (10) can be started to washing with water or other solution acidified (for example with sulfuric acid or phosphoric acid), allowing the recovery of nitrogen in the form of sulfate salts ammonium or ammonium phosphate.

The substrate with lower content of ammoniacal nitrogen can then be initiated to anaerobic digestion for production of biogas, being now reduced the initial toxicity that was due to ammonia.

In fact in the process object of the present application, the nitrogen content of manure in input to the process undergoes a reduction in mass downstream of the hydrolysis and the subsequent heat treatment and therefore before a possible anaerobic digestion. It should be noted that a high concentration of ammonia nitrogen can result in toxic effects to the methanogenic biomass.

## Claims

1. Pre-treatment process of organic substrates with a high organic nitrogen content such as the manure produced by laying hens or other poultry (1), for the reduction of the organic nitrogen content prior to complete anaerobic digestion treatment, the process being **characterized by** the following steps:
a) mixing (2) and feeding of the substrate (4) to the reactor (6);
b) heating of the organic substrate at a controlled temperature between 20 and 55° C to perform a hydrolysis and acidogenesis phase (6) until a minimum of 25% of total nitrogen is mineralized into ammonia nitrogen while the release and accumulation of carbon dioxide CO₂ and volatile fatty acids in the substrate is facilitated;
c) a second thermal treatment (7) of the substrate is performed at the end of the hydrolysis and acidogenesis phase consisting in its heating up to a temperature between 65 and 95° C, thereby reducing the CO₂ solubility and then stripping it (8) in order to start alkalinization of the substrate by increasing the PH parameter to values equal or higher than 9, thus shifting the ammonia chemical equilibrium from the aqueous phase of NH₄0H to the gaseous phase of NH₃,
and finally:
d) stripping of nitrogen NH₃ (9) and its removal (10) from the substrate.

2. Process according to claim 1, **characterized in that** after completion of the hydrolysis/ acidogenesis phase (6), time can be interposed before starting the thermal treatment (7-8) and nitrogen stripping phase (9).

3. Process according to claims 1 and 2, **characterized in that** the substrate(1) is livestock manure, or a mixture of different livestock manure, or other substrates with a concentration of organic nitrogen higher than 30,0 kg Norg per ton of dry matter, such as poultry manure, which has more than 40 kg Ntot/ton of dry matter.

4. Process according to claims 1 to 3, **characterized in that** the substrate (1) is mixed (2) with water and/or digestate (3) with the aim of obtaining a pumpable mixture (4) and/or favour the hydrolysis/acidogenesis phase (6).

5. Process according to claims 1 to 4, **characterized in that** solids and/or floating materials (5) can be removed from the substrate.

6. Process according to claims 1 to 5, **characterized in that** each step of the pre-treatment (2-6-7/8-9) can be in continuous or in batch.

7. Process according to claims 1 to 6, **characterized in that** the substrate treated in phase (6) can be accumulated before going to phase (7/8)

8. Process according to claims 1 to 7, **characterized in that** water and/or sewage and/or digestate and/or their mixture (3) may be added at each different step (2-6-7/8-9).

9. Process according to claims 1 to- 8, **characterized in that** the fully pretreated substrate is subsequently used as biomass for the biogas production through a complete anaerobic digestion process.

10. Device for the pre-treatment of organic substrates prior to complete anaerobic digestion following the process of one or more of the previous claims, comprising:
- means to mix and feed the substrate;
- one or more reactors connected in series to perform hydrolysis and thermal treatment steps;
- means for stripping nitrogen NH₃ from the substrate after the thermal treatment is completed.

## Patentansprüche

1. Vorbehandlungsverfahren organischer Substanzen mit einem hohen organischen Stickstoffgehalt, wie etwa durch Legehennen oder anderes Geflügel (1) erzeugter Dung, für die Verringerung des organischen Stickstoffgehalts vor vollständig anaerober Zersetzungsbehandlung, wobei das Verfahren durch die folgenden Schritte gekennzeichnet ist:
a) Mischen (2) und Zuführen des Substrats (4) zu dem Reaktor (6) ;
b) Erwärmen des organischen Substrats bei einer geregelten Temperatur zwischen 20 und 55 °C zum Durchführen einer Hydrolyse- und Acidogenesephase (6), bis ein Minimum von 25 % des Gesamtstickstoffs zu Ammoniak-Stickstoff mineralisiert ist, während die Freisetzung und Ansammlung von Kohlenstoffdioxid CO2 und flüchtigen Fettsäuren in dem Substrat erleichtert wird;
c) eine zweite Wärmebehandlung (7) des Substrats wird an dem Ende der Hydrolyse-und Acidogenesephase durchgeführt, bestehend aus seiner Erwärmung auf eine Temperatur zwischen 65 und 95 °C, sodass die CO2-Löslichkeit verringert wird und es dann ausgetrieben wird (8), um eine Alkalisierung des Substrats durch Steigern des PH-Parameters auf Werte gleich oder höher als 9 zu beginnen, sodass das chemische Gleichgewicht des Ammoniaks von der wässrigen Phase aus NH4OH zu der Gasphase aus NH3 verschoben wird,
und schließlich:
d) Austreiben von Stickstoff NH3 (9) und seine Entfernung (10) aus dem Substrat.

2. Verfahren gemäß Anspruch 1, **dadurch gekennzeichnet, dass** nach Abschluss der Hydrolyse-/Acidogenesephase (6) vor Beginn der Wärmebehandlung (7-8) und Stickstoffaustreibungsphase (9) Zeit eingeschoben werden kann.

3. Verfahren gemäß Ansprüchen 1 und 2, **dadurch gekennzeichnet, dass** das Substrat (1) Viehdung, oder eine Mischung aus unterschiedlichem Viehdung oder andere Substrate mit einer Konzentration an organischem Stickstoff höher als 30,0 kg Norg pro Tonne Trockensubstanz, wie etwa Geflügeldung, der mehr als 40 kg Ntot/Tonne an Trockensubstanz aufweist, ist.

4. Verfahren gemäß Ansprüchen 1 bis 3, **dadurch gekennzeichnet, dass** das Substrat (1) mit Wasser und/oder Gärrest (3) gemischt wird (2), mit dem Ziel, eine pumpbare Mischung (4) zu erhalten und/oder die Hydrolyse-/Acidogenesephase (6) zu begünstigen.

5. Verfahren gemäß Ansprüchen 1 bis 4, **dadurch gekennzeichnet, dass** Feststoffe und/oder Schwebematerialien (5) aus dem Substrat entfernt werden können.

6. Verfahren gemäß Ansprüchen 1 bis 5, **dadurch gekennzeichnet, dass** jeder Schritt der Vorbehandlung (2-6-7/8-9) kontinuierlich oder chargenweise sein kann.

7. Verfahren gemäß Ansprüchen 1 bis 6, **dadurch gekennzeichnet, dass** das Substrat, das in Phase (6) behandelt wird, angesammelt werden kann, bevor es zu Phase (7/8) geht.

8. Verfahren gemäß Ansprüchen 1 bis 7, **dadurch gekennzeichnet, dass** Wasser und/oder Abwasser und/oder Gärrest und/oder deren Mischung (3) in jedem unterschiedlichen Schritt (2-6-7/8-9) zugesetzt werden kann.

9. Verfahren gemäß Ansprüchen 1 bis 8, **dadurch gekennzeichnet, dass** das vollständig vorbehandelte Substrat anschließend als Biomasse für die Biogasproduktion durch ein vollständig anaerobes Zersetzungsverfahren verwendet wird.

10. Vorrichtung für die Vorbehandlung organischer Substanzen vor vollständig anaerober Zersetzung nach dem Verfahren eines oder mehrerer der vorhergehenden Ansprüche, das umfasst:
- Mittel zum Mischen und Zuführen des Substrats;
- einen oder mehrere Reaktoren, die in Reihe geschaltet sind, um Hydrolyse- und Wärmebehandlungsschritte auszuführen;
- Mittel zum Austreiben von Stickstoff NH3 aus dem Substrat, nachdem die Wärmebehandlung abgeschlossen wurde.

## Revendications

1. Procédé de prétraitement de substrats organiques ayant une teneur élevée en azote organique tel que le fumier produit par des poules pondeuses ou autres volailles (1), permettant la réduction de la teneur en azote organique avant un traitement de digestion anaérobie complet, ce procédé étant **caractérisé par** les étapes suivantes consistant à :
a) mélanger (2) et introduire le substrat (4) dans un réacteur (6),
b) chauffer le substrat organique à une température contrôlée comprise entre 20 et 55°C pour effectuer une étape d'hydrolyse et d'acidogènèse (6) jusqu'à ce qu'un minimum de 25% de l'azote total soit minéralisé en azote ammoniacal en facilitant le dégagement et l'accumulation de dioxyde de carbone CO₂ et acides gras volatiles dans le substrat,
c) effectuer un second traitement thermique (7) du substrat à la fin de l'étape d'hydrolyse et d'acidogènèse constitué par un chauffage jusqu'à une température comprise entre 65 et 95°C de façon à réduire la solubilité du CO₂ à le dégager en (8) pour commencer l'alcalinisation du substrat en augmentant le pH à des valeurs égales ou supérieures à 9, en décalant ainsi l'équilibre chimique de l'ammoniac entre la phase NH₄OH aqueuse et la phase NH₃ gazeux,
et finalement,
d) dégager le NH₃ (9) et l'extraire (10) du substrat.

2. Procédé conforme à la revendication 1,
**caractérisé en ce qu'**
après l'achèvement de l'étape d'hydrolyse/d'acidogènèse (6) il peut y avoir une temporisation avant le début du traitement thermique (7-8) et la phase de dégagement de l'azote (9).

3. Procédé conforme aux revendications 1 et 2,
**caractérisé en ce que**
le substrat (1) est un fumier de bétail ou un mélange de différents fumiers de bétail ou d'autres substrats ayant une concentration en azote organique supérieure à 30,0 kg de Norg par tonne de matériau sec, tel qu'un fumier de volaille qui renferme plus de 40 kg de Ntot par tonne de matériau sec.

4. Procédé conforme aux revendications 1 à 3,
**caractérisé en ce que**
le substrat (1) est mélangé (2) avec de l'eau et/ou des produits de digestion (3) pour obtenir un mélange pompable (4) et/ou favoriser l'étape d'hydrolyse/acidogènèse (6).

5. Procédé conforme aux revendications 1 à 4,
**caractérisé en ce que**
des matériaux solides et/ou flottants (5) peuvent être extraits du substrat.

6. Procédé conforme aux revendications 1 à 5,
**caractérisé en ce que**
chacune des étapes du prétraitement (2-6-7/8-9) peut être effectué en continu ou par bâchées.

7. Procédé conforme aux revendications 1 à 6,
**caractérisé en ce que**
le substrat traité lors de l'étape (6) peut être accumulé avant le début de l'étape (7/8).

8. Procédé conforme aux revendications 1 à 7,
**caractérisé en ce que**
de l'eau et/ou des boissons et/ou des produits de digestion et/ou leurs mélanges (3) peut(vent) être ajouté(es) lors de chaque étape (2-6-7/8-9).

9. Procédé conforme aux revendications 1 à 8,
**caractérisé en ce que**
le substrat totalement prétraité est ensuite utilisé en tant que bio masse pour la production de bio gaz par un procédé de digestion anaérobie complète.

10. Dispositif de prétraitement de substrats organiques avant digestion anaérobie complète permettant la mise en oeuvre du procédé conforme à au moins l'une des revendications précédentes comprenant :
- des moyens permettant de mélanger et d'alimenter le substrat,
- un réacteur ou plusieurs réacteurs branchés en série pour permettre d'effectuer les étapes d'hydrolyse et de traitements thermiques,
- des moyens d'extraction de l'azote sous forme de NH₃ du substrat après que le traitement thermique ait été achevé.
